# EUROPEAN PATENT APPLICATION

(11) **EP 1 375 653 A1**
(43) Date of publication of application: **02.01.2004**
(21) Application number: 03254126.0
(22) Date of filing: 27.06.2003
(51) Int. Cl.: C12N 13/00, C12N 1/16, A61K 41/00, A61K 35/72, A23L 1/28, A61P 25/00

(54) **Dietary supplements for regulating the central nervous system**

(30) Priority: 28.06.2002 US 186505
(71) Applicant: Ultra Biotech Limited, Douglas IM1 1SA, Isle of Man (GB)
(72) Inventor: Cheung, Ling Yuk, Hong Kong (CN)
(74) Representative: Kyle, Diana

(57) **Abstract**

Compositions comprising a plurality of yeast cells, wherein said plurality of yeast cells have been cultured in the presence of an alternating electric field having a specific frequency and a specific field strength for a period of time sufficient to increase the capability of said plurality of yeast cells to regulate the central nervous system. Also included are methods of making such compositions.

## Description

### FIELD OF THE INVENTION

The invention relates to compositions that benefit the central nervous system and can be taken as dietary supplements. The compositions comprise yeast cells obtainable by growth in electromagnetic fields with specific frequencies and field strengths.

### BACKGROUND OF THE INVENTION

Researchers have been trying to develop drugs that are effective in treating diseases such as Alzheimer's disease and diseases related to dementia, depression and neurasthemia, which involve the central nervous system. Although there have been several drugs on the market in this area, these drugs only delay the progression of the diseases and do not provide a cure. Furthermore, the drugs are often small molecule inhibitors that produce side effects.

### SUMMARY OF THE INVENTION

The composition of the invention assists in the recovery of Alzheimer's disease and diseases related to dementia, depression and. neurasthemia. The composition also assists in the recovery of brain damage and brain metabolism blockade and can be taken as dietary supplements in the form of health drinks or pills.

This invention embraces a composition comprising a plurality of yeast cells that have been cultured in an alternating electric field having a frequency in the range of about 13050 to 13150 MHZ, and a field strength in the range of about 20 to 400 mV/cm. In one embodiment, the frequency is in the range of 13100-13150 MHZ. In another embodiment, the field strength is in the range of 50-300 mV/cm. The yeast cells are cultured in the alternating electric field for a period of time sufficient to increase the capability of said plurality of yeast cells to regulate the central nervous system of a mammal as compared to unactivated yeast cells. In one embodiment, the composition comprising the activated yeast cells increases the amount of met-enkaphalin (MEK) or leu-enkaphalin (LEK) in the brain tissue or brain cell of a mammal. In another embodiment, the composition comprising the activated yeast cells has a calming effect on the central nervous system. In yet another embodiment, the composition substantially increases the low frequency electroencephalogram(EEG) power spectra of the brain of a mammal. In one embodiment, the frequency and/or the field strength of the alternating electric field can be altered within the aforementioned ranges during said period of time. In other words, the yeast cells can be exposed to a series of electromagnetic fields. An exemplary period of time is about 40 to 150 hours. In one embodiment, the period of time is 60-90 hours. Included within this invention are also methods of making these compositions.

Yeast cells that can be included in this composition can all be obtained from the China General Microbiological Culture Collection Center ("CGMCC"), a depository recognized under the Budapest Treaty (China Committee for Culture Collection of Microorganisms, Institute of Microbiology, Chinese Academy of Sciences, Haidian, P.O. BOX 2714, Beijing, 100080, China). Useful yeast species include, but are not limited to *Schizosaccharomyces pombe, Saccharmyces sake, Saccharomyces urarum, Saccharomyces rouxii, Saccharomyces carlsbergensis, Rhodotorula aurantiaca* and *Saccharomyces cerevisiae.* In one embodiment, the yeast species is *Saccharomyces carlsbergensis Hansen or Saccharomyces cerevisiae Hansen.* For instance, the yeast cells can be of the strain *Saccharomyces carlsbergensis Hansen* AS2.443. In one embodiment, the yeast cells are from the strains selected from the group consisting of AS2.501, AS2.502, AS2.503, AS2.504, AS2.535, AS2.558, AS2.560, AS2.561, AS2.443 and AS2.562. Other useful yeast species are illustrated in Table 1.

As used herein, "substantially increase" refers to an increase of more than 3 fold. In one embodiment, the increase is 5 fold.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention. All publications and other references mentioned herein are incorporated by reference in their entirety. In case of conflict, the present specification, including definitions, will control. The materials, methods, and examples are illustrative only and not intended to be limiting. Throughout this specification and claims, the word "comprise," or variations such as "comprises" or "comprising" will be understood to imply the inclusion of a stated integer or group of integers but not the exclusion of any other integer or group of integers.

Other features and advantages of the invention will be apparent from the following detailed description, and from the claims.

### BRIEF DESCRIPTION OF THE DRAWINGS

Fig. 1 is a schematic diagram showing an exemplary apparatus for activating yeast cells using electromagnetic fields. 1: yeast culture; 2: container; 3: power supply.
Fig. 2 is a schematic diagram showing an exemplary apparatus for making yeast compositions of the invention. The apparatus comprises a signal generator and interconnected containers 1, 2 and 3.

### DETAILED DESCRIPTION OF THE INVENTION

This invention is based on the discovery that certain yeast strains can be activated by electromagnetic fields ("EMF") having specific frequencies and field strengths to produce agents useful in regulating the central nervous system. Yeast compositions comprising activated yeast cells can be used as dietary supplements in the form of health drinks or pills.

In certain embodiments, the yeast compositions of this invention increase the levels of MEK, LEK or both in the brain tissue or brain cell of a mammal. In another embodiment, the yeast compositions of this invention have a calming effect on the central nervous system of a mammal. In yet another embodiment, the yeast compositions of this invention substantially increase the low frequency EEG power spectra of the brain of a mammal. In one embodiment, the mammal is human. Compositions comprising the activated yeast cells are useful in regulating the central nervous system.

Since the activated yeast cells contained in these yeast compositions have been cultured to endure acidic conditions of pH 2.5-4.2, the compositions are stable in the stomach and can pass on to the intestines. Once in the intestines, the yeast cells are ruptured by various digestive enzymes, and agents for regulating the central nervous system are released and readily absorbed.

Without being bound by any theory or mechanism, the inventor believes that EMFs activate or enhance the expression of a gene or a set of genes in the yeast cells such that the yeast cells become active or more efficient in performing certain metabolic activities which lead to the desired result of regulating the central nervous system.

### I. Yeast Strains Useful in the Invention

The types of yeasts useful in this invention include, but are not limited to, yeasts of the genera *Saccharomyces Schizosaccharomyces,* and *Rhodotorula.*

Exemplary species within the above-listed genera include, but are not limited to, the species illustrated in Table 1. Yeast strains useful in this invention can be obtained from laboratory cultures, or from publically accessible culture depositories, such as CGMCC and the American Type Culture Collection, 10801 University Boulevard, Manassas, VA 20110-2209. Non-limiting examples of useful strains (with the accession numbers of CGMCC) are illustrated in Table 1. In general, yeast strains preferred in this invention are those used for fermentation in the food and wine industries. As a result, compositions containing these yeast cells are safe for human consumption.

Although it is preferred, the preparation of the yeast compositions of this invention is not limited to starting with a pure strain of yeast. A yeast composition of the invention may be produced by culturing a mixture of yeast cells of different species or strains.

**Table 1**

| Exemplary Yeast Strains | | | | |
|---|---|---|---|---|
| | *Saccharomyces cerevisiae* Hansen | | | |
| ACCC2034 | ACCC2035 | ACCC2036 | ACCC2037 | ACCC2038 |
| ACCC2039 | ACCC2040 | ACCC2041 | ACCC2042 | AS2. 1 |
| AS2. 4 | AS2. 11 | AS2. 14 | AS2. 16 | AS2. 56 |
| AS2. 69 | AS2. 70 | AS2. 93 | AS2. 98 | AS2. 101 |
| AS2. 109 | AS2. 110 | AS2. 112 | AS2. 139 | AS2. 173 |
| AS2. 174 | AS2. 182 | AS2. 196 | AS2. 242 | AS2. 336 |
| AS2. 346 | AS2. 369 | AS2. 374 | AS2. 375 | AS2. 379 |
| AS2. 380 | AS2. 382 | AS2. 390 | AS2. 393 | AS2. 395 |
| AS2. 396 | AS2.397 | AS2. 398 | AS2. 399 | AS2. 400 |
| AS2. 406 | AS2. 408 | AS2. 409 | AS2. 413 | AS2. 414 |
| AS2. 415 | AS2. 416 | AS2. 422 | AS2. 423 | AS2. 430 |
| AS2. 431 | AS2. 432 | AS2. 451 | AS2. 452 | AS2. 453 |
| AS2. 458 | AS2. 460 | AS2. 463 | AS2. 467 | AS2. 486 |
| AS2. 501 | AS2. 502 | AS2. 503 | AS2. 504 | AS2. 516 |
| AS2. 535 | AS2. 536 | AS2. 558 | AS2. 560 | AS2. 561 |
| AS2. 562 | AS2. 576 | AS2. 593 | AS2. 594 | AS2. 614 |
| AS2. 620 | AS2.628 | AS2. 631 | AS2. 666 | AS2. 982 |
| AS2. 1190 | AS2. 1364 | AS2. 1396 | IFFI1001 | IFFI1002 |
| IFFI1005 | IFFI1006 | IFFI1008 | IFFI1009 | IFFI1010 |
| IFFI1012 | IFFI1021 | IFFI1027 | IFFI1037 | IFFI1042 |
| IFFI1043 | IFFI1045 | IFFI1048 | IFFI1049 | IFFI1050 |
| IFFI1052 | IFFI1059 | IFFI1060 | IFFI1062 | IFFI1063 |
| IFFI1202 | IFFI1203 | IFFI1206 | IFFI1209 | IFFI1210 |
| IFFI1211 | IFFI1212 | IFFI1213 | IFFI1214 | IFFI1215 |
| IFFI1220 | IFFI1221 | IFFI1224 | IFFI1247 | IFFI1248 |
| IFFI1251 | IFFI1270 | IFFI1277 | IFFI1287 | IFFI1289 |
| IFFI1290 | IFFI1291 | IFFI1292 | IFFI1293 | IFFI1297 |
| IFFI1300 | IFFI1301 | IFFI1302 | IFFI1307 | IFFI1308 |
| IFFI1309 | IFFI1310 | IFFI1311 | IFFI1331 | IFFI1335 |
| IFFI1336 | IFFI1337 | IFFI1338 | IFFI1339 | IFFI1340 |
| IFFI1345 | IFFI1348 | IFFI1396 | IFFI1397 | IFFI1399 |
| IFFI1411 | IFFI1413 | IFFI1441 | IFFI1443 | |

| | *Saccharomyces cerevisiae* Hansen Var. ellipsoideus (Hansen) Dekker | | | |
|---|---|---|---|---|
| ACCC2043 | AS2.2 | AS2.3 | AS2.8 | AS2.53 |
| AS2.163 | AS2.168 | AS2.483 | AS2.541 | AS2.559 |
| AS2.606 | AS2.607 | AS2.611 | AS2.612 | |

| | *Saccharomyces chevalieri* Guilliermond | | | |
|---|---|---|---|---|
| AS2.131 | AS2.213 | | | |

| | *Saccharomyces delbrueckii* | | | |
|---|---|---|---|---|
| AS2.285 | | | | |

| | *Saccharomyces delbrueckii* Lindner ver. mongolicus (Saito) Lodder et van Rij | | | |
|---|---|---|---|---|
| AS2.209 | AS2.1157 | | | |

| | *Saccharomyces exiguous* Hansen | | | |
|---|---|---|---|---|
| AS2.349 | AS2.1158 | | | |

| | *Saccharomyces fermentati* (Saito) Lodder et van Rij | | | |
|---|---|---|---|---|
| AS2.286 | AS2.343 | | | |

| | *Saccharomyces logos* van laer et Denamur ex Jorgensen | | | |
|---|---|---|---|---|
| AS2.156 | AS2.327 | AS2.335 | | |

| | *Saccharomyces mellis* (Fabian et Quinet) Lodder et kreger van Rij | | | |
|---|---|---|---|---|
| AS2.195 | | | | |

| | *Saccharomyces mellis* Microellipsoides Osterwalder | | | |
|---|---|---|---|---|
| AS2.699 | | | | |

| | *Saccharomyces oviformis* Osteralder | | | |
|---|---|---|---|---|
| AS2.100 | | | | |

| | *Saccharomyces rosei* (Guilliermond) Lodder et Kreger van Rij | | | |
|---|---|---|---|---|
| AS2.287 | | | | |

| | *Saccharomyces rouxii* Boutroux | | | |
|---|---|---|---|---|
| AS2.178 | AS2.180 | AS2.370 | AS2.371 | |

| | *Saccharomyces sake* Yabe | | | |
|---|---|---|---|---|
| ACCC2045 | | | | |

| | *Candida arborea* | | | |
|---|---|---|---|---|
| AS2.566 | | | | |

| | *Candida lambica* (Lindner et Genoud) van. Uden et Buckley | | | |
|---|---|---|---|---|
| AS2.1182 | | | | |

| | *Candida krusei* (Castellani) Berkhout | | | |
|---|---|---|---|---|
| AS2.1045 | | | | |

| | *Candida lipolytica* (Harrison) Diddens et Lodder | | | |
|---|---|---|---|---|
| AS2.1207 | AS2.1216 | AS2.1220 | AS2.1379 | AS2.1398 |
| AS2.1399 | AS2.1400 | | | |

| | *Candida parapsilosis* (Ashford) Langeron et Talice Var. intermedia Van Rij et Verona | | | |
|---|---|---|---|---|
| AS2.491 | | | | |

| | *Candida parapsilosis* (Ashford) Langeron et Talice | | | |
|---|---|---|---|---|
| AS2.590 | | | | |

| | *Candida pulcherrima* (Lindner) Windisch | | | |
|---|---|---|---|---|
| AS2.492 | | | | |

| | *Candida rugousa* (Anderson) Diddens et Lodder | | | |
|---|---|---|---|---|
| AS2.511 | AS2.1367 | AS2.1369 | AS2.1372 | AS2.1373 |
| AS2.1377 | AS2.1378 | AS2.1384 | | |

| | *Candida tropicalis* (Castellani) Berkhout | | | |
|---|---|---|---|---|
| ACCC2004 | ACCC2005 | ACCC2006 | AS2.164 | AS2.402 |
| AS2.564 | AS2.565 | AS2.567 | AS2.568 | AS2.617 |
| AS2.637 | AS2.1387 | AS2.1397 | | |

| | *Candida utilis* Henneberg Lodder et Kreger Van Rij | | | |
|---|---|---|---|---|
| AS2.120 | AS2.281 | AS2.1180 | | |

| | *Crebrothecium ashbyii* (Guillermond) Routein *(Eremothecium ashbyii* Guilliermond) | | | |
|---|---|---|---|---|
| AS2.481 | AS2.482 | AS2.1197 | | |

| | *Geotrichum candidum* Link | | | |
|---|---|---|---|---|
| ACCC2016 | AS2.361 | AS2.498 | AS2.616 | AS2.1035 |
| AS2.1062 | AS2.1080 | AS2.1132 | AS2.1175 | AS2.1183 |

| | *Hansenula anomala* (Hansen)H et P sydow | | | |
|---|---|---|---|---|
| ACCC2018 | AS2.294 | AS2.295 | AS2.296 | AS2.297 |
| AS2.298 | AS2.299 | AS2.300 | AS2.302 | AS2.338 |
| AS2.339 | AS2.340 | AS2.341 | AS2.470 | AS2.592 |
| AS2.641 | AS2.642 | AS2.782 | AS2.635 | AS2.794 |

| | *Hansenula arabitolgens* Fang | | | |
|---|---|---|---|---|
| AS2.887 | | | | |

| | *Hansenula jadinii* (A. et R Sartory Weill et Meyer) Wickerham | | | |
|---|---|---|---|---|
| ACCC2019 | | | | |

| | *Hansenula saturnus* (Klocker) H et P sydow | | | |
|---|---|---|---|---|
| ACCC2020 | | | | |

| | *Hansenula schneggii* (Weber ) Dekker | | | |
|---|---|---|---|---|
| AS2.304 | | | | |

| | *Hansenula subpelliculosa* Bedford | | | |
|---|---|---|---|---|
| AS2.740 | AS2.760 | AS2.761 | AS2.770 | AS2.783 |
| AS2.790 | AS2.798 | AS2.866 | | |

| | *Kloeckera apiculata* (Reess emend. Klocker) Janke | | | |
|---|---|---|---|---|
| ACCC2022 | ACCC2023 | AS2.197 | AS2.496 | AS2.714 |
| ACCC2021 | AS2.711 | | | |

| | *Lipomycess starkeyi* Lodder et van Rij | | | |
|---|---|---|---|---|
| AS2.1390 | ACCC2024 | | | |

| | *Pichia farinosa* (Lindner) Hansen | | | |
|---|---|---|---|---|
| ACCC2025 | ACCC2026 | AS2.86 | AS2.87 | AS2.705 |
| AS2.803 | | | | |

| | *Pichia membranaefaciens* Hansen | | | |
|---|---|---|---|---|
| ACCC2027 | AS2.89 | AS2.661 | AS2.1039 | |

| | *Rhodosporidium toruloides* Banno | | | |
|---|---|---|---|---|
| ACCC2028 | | | | |

| | *Rhodotorula glutinis* (Fresenius) Harrison | | | |
|---|---|---|---|---|
| AS2.2029 | AS2.280 | ACCC2030 | AS2.102 | AS2.107 |
| AS2.278 | AS2.499 | AS2.694 | AS2.703 | AS2.704 |
| AS2.1146 | | | | |

| | *Rhodotorula minuta* (Saito) Harrison | | | |
|---|---|---|---|---|
| AS2.277 | | | | |

| | *Rhodotorula rubar* (Demme) Lodder | | | |
|---|---|---|---|---|
| AS2.21 | AS2.22 | AS2.103 | AS2.105 | AS2.108 |
| AS2.140 | AS2.166 | AS2.167 | AS2.272 | AS2.279 |
| AS2.282 | ACCC2031 | | | |

| | *Rhodotorula aurantiaca* (Saito) Lodder | | | |
|---|---|---|---|---|
| AS2.102 | AS2.107 | AS2.278 | AS2.499 | AS2.694 |
| AS2.703 | AS2.704 | AS2.1146 | | |

| | *Saccharomyces carlsbergensis* Hansen | | | |
|---|---|---|---|---|
| AS2.113 | ACCC2032 | ACCC2033 | AS2.312 | AS2.116 |
| AS2.118 | AS2.121 | AS2.132 | AS2.162 | AS2.189 |
| AS2.200 | AS2.216 | AS2.265 | AS2.377 | AS2.417 |
| AS2.420 | AS2.440 | AS2.441 | AS2.443 | AS2.444 |
| AS2.459 | AS2.595 | AS2.605 | AS2.638 | AS2.742 |
| AS2.745 | AS2.748 | AS2.1042 | | |

| | *Saccharomyces uvarum* Beijer | | | |
|---|---|---|---|---|
| IFFI1023 | IFFI1032 | IFFI1036 | IFFI1044 | IFFI1072 |
| IFFI1205 | IFFI1207 | | | |

| | *Saccharomyces willianus* Saccardo | | | |
|---|---|---|---|---|
| AS2.5 | AS2.7 | AS2.119 | AS2.152 | AS2.293 |
| AS2.381 | AS2.392 | AS2.434 | AS2.614 | AS2.1189 |

| | *Saccharomyces sp.* | | | |
|---|---|---|---|---|
| AS2.311 | | | | |

| | *Saccharomycodes ludwigii* Hansen | | | |
|---|---|---|---|---|
| ACCC2044 | AS2.243 | AS2.508 | | |

| | *Saccharomycodes sinenses* Yue | | | |
|---|---|---|---|---|
| AS2.1395 | | | | |

| | *Schizosaccharomyces octosporus* Beijerinck | | | |
|---|---|---|---|---|
| ACCC2046 | AS2.1148 | | | |

| | *Schizosaccharomyces pombe* Lindner | | | |
|---|---|---|---|---|
| ACCC2047 | ACCC2048 | AS2.214 | AS2.248 | AS2.249 |
| AS2.255 | AS2.257 | AS2.259 | AS2.260 | AS2.274 |
| AS2.994 | AS2.1043 | AS2.1149 | AS2.1178 | IFFI1056 |

| | *Sporobolomyces roseus* Kluyver et van Niel | | | |
|---|---|---|---|---|
| ACCC2049 | ACCC2050 | AS2.19 | AS2.962 | AS2.1036 |
| ACCC2051 | AS2.261 | AS2.262 | | |

| | *Torulopsis candida* (Saito) Lodder | | | |
|---|---|---|---|---|
| AS2.270 | ACCC2052 | | | |

| | *Torulopsis famta* (Harrison) Lodder et van Rij | | | |
|---|---|---|---|---|
| ACCC2053 | AS2.685 | | | |

| | *Torulopsis globosa* (Olson et Hammer) Lodder et van Rij | | | |
|---|---|---|---|---|
| ACCC2054 | AS2.202 | | | |

| | *Torulopsis inconspicua* Lodder et Kreger van Rij | | | |
|---|---|---|---|---|
| AS2.75 | | | | |

| | *Trichosporon behrendii* Lodder et Kreger van Rij | | | |
|---|---|---|---|---|
| ACCC2056 | AS2.1193 | | | |

| | *Trichosporon capitatum* Diddens et Lodder | | | |
|---|---|---|---|---|
| ACCC2056 | AS2.1385 | | | |

| | *Trichosporon cutaneum* (de Beurm et al.) Ota | | | |
|---|---|---|---|---|
| ACCC2057 | AS2.25 | AS2.570 | AS2.571 | AS2.1374 |

| | *Wickerhamia fluorescens* (Soneda) Soneda | | | |
|---|---|---|---|---|
| ACCC2058 | AS2-1388 | | | |

### II. Application of Electromagnetic Fields

An electromagnetic field useful in this invention can be generated and applied by various means well known in the art. For instance, the EMF can be generated by applying an alternating electric field or an oscillating magnetic field.

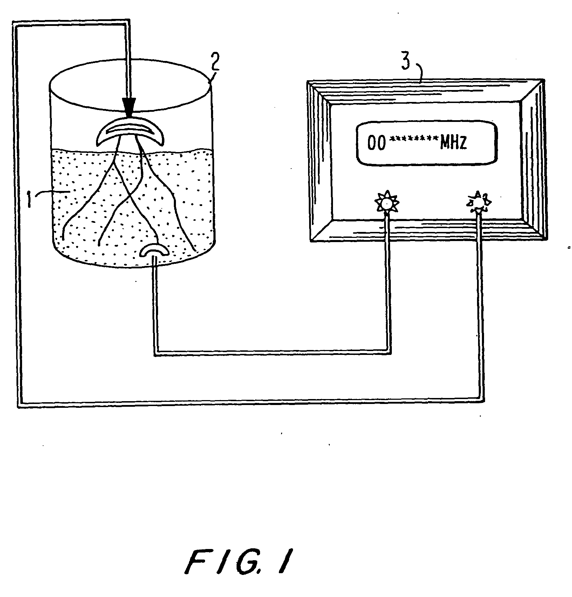

Alternating electric fields can be applied to cell cultures through electrodes in direct contact with the culture medium, or through electromagnetic induction. See, e.g., Fig. 1. Relatively high electric fields in the medium can be generated using a method in which the electrodes are in contact with the medium. Care must be taken to prevent electrolysis at the electrodes from introducing undesired ions into the culture and to prevent contact resistance, bubbles, or other features of electrolysis from dropping the field level below that intended. Electrodes should be matched to their environment, for example, using Ag-AgCl electrodes in solutions rich in chloride ions, and run at as low a voltage as possible. For general review, see Goodman et al., *Effects of EMF on Molecules and Cells,* International Review of Cytology, A Survey of Cell Biology, Vol. 158, Academic Press, 1995.

The EMFs useful in this invention can also be generated by applying an oscillating magnetic field. An oscillating magnetic field can be generated by oscillating electric currents going through Helmholtz coils. Such a magnetic field in turn induces an electric field.

The frequencies of EMFs useful in this invention range from about 13050 MHZ to 13150 MHZ. Exemplary frequencies include 13103, 13107, 13113, 13119 and 13125 MHZ. The field strength of the electric field useful in this invention ranges from about 20 to 400 mV/cm (e.g., 60-100, 190-220, 240-280, 270-290, 300-330 or 350-380 mV/cm). Exemplary field strengths include 73, 94, 202, 206, 257, 272, 273, 277, 282, 284, 303 and 372 mV/cm.

When a series of EMFs are applied to a yeast culture, the yeast culture can remain in the same container while the same set of EMF generator and emitters is used to change the frequency and/or field strength. The EMFs in the series can each have a different frequency or a different field strength; or a different frequency and a different field strength. Such frequencies and field strengths are preferably within the above-described ranges. Although any practical number of EMFs can be used in a series, it may be preferred that the yeast culture be exposed to a total of 2, 3, 4, 5, 6, 7, 8, 9 or 10 EMFs in a series.

Although the yeast cells can be activated after even a few hours of culturing in the presence of an EMF, it may be preferred that the compositions comprising activated yeast cells be allowed to multiply and grow in the presence of the EMF(s) for a total of 40-150 hours, preferably, 60-90 hours.

Fig. 1 illustrates an exemplary apparatus for generating alternating electric fields. An electric field of a desired frequency and intensity can be generated by an AC source (3) capable of generating an alternating electric field, preferably in a sinusoidal wave form, in the frequency range of 5 to 20,000 MHZ. Signal generators capable of generating signals with a narrower frequency range can also be used. If desired, a signal amplifier can also be used to increase the output. The culture container (2) can be made from a non-conductive material, e.g., glass, plastic or ceramic. The cable connecting the culture container (2) and the signal generator (3) is preferably a high frequency coaxial cable with a transmission frequency of at least 30 GHz.

The alternating electric field can be applied to the culture by a variety of means, including placing the yeast culture (1) in close proximity to the signal emitters such as a metal wire or tube capable of transmitting EMFs. The metal wire or tube can be made of red copper, and be placed inside the container (2), reaching as deep as 3-30 cm. For example, if the fluid in the container (2) has a depth of 15-20 cm, 20-30 cm, 30-50 cm, 50-70 cm, 70-100 cm, 100-150 cm or 150-200 cm, the metal wire can be 3-5 cm, 5-7 cm, 7-10 cm, 10-15 cm, 15-20 cm, 20-30 cm and 25-30 cm from the bottom of the container (2), respectively. The number of metal wires/tubes used can be from 1 to 10 (e.g., 2 to 3). It is recommended, though not mandated, that for a culture having a volume up to 10 L, metal wires/tubes having a diameter of 0.5 to 2 mm be used. For a culture having a volume of 10-100 L, metal wires/tubes having a diameter of 3 to 5 mm can be used. For a culture having a volume of 100-1000 L, metal wires/tubes having a diameter of 6 to 15 mm can be used. For a culture having a volume greater than 1000 L, metal wires/tubes having a diameter of 20-25 mm can be used.

In one embodiment, the electric field is applied by electrodes submerged in the culture (1). In this embodiment, one of the electrodes can be a metal plate placed on the bottom of the container (2), and the other electrode can comprise a plurality of electrode wires evenly distributed in the culture (1) so as to achieve even distribution of the electric field energy. The number of electrode wires used depends on the volume of the culture as well as the diameter of the wires.

### III. Culture Media

Culture media useful in this invention contain sources of nutrients assimilatable by yeast cells. Complex carbon-containing substances in a suitable form (e.g., carbohydrates such as sucrose, glucose, dextrose, maltose, starch and xylosel; or mannitol) can be the carbon sources for yeast cells. The exact quantity of the carbon sources can be adjusted in accordance with the other ingredients of the medium. In general, the amount of carbon-containing substances varies between about 0.5% and 10% by weight of the medium, and preferably between about 1% and 5%, most preferably between about 1.5-2.5%. Vitamins can also be added to the medium, for example, Vitamin E, H and B12. Among the inorganic salts which can be added to a laboratory culture medium are the customary salts capable of yielding sodium, potassium, calcium, phosphate, sulfate, carbonate, and like ions. Non-limiting examples of nutrient inorganic salts are (NH₄)₂HPO₄, CaCO₃, KH₂PO₄, K₂HPO₄, MgSO₄, NaCl, and CaSO₄.

### IV. Electromagnetic Activation of Yeast Cells

To activate or enhance the innate ability of yeast cells to produce agents that are useful in regulating the central nervous system, these cells can be cultured in an appropriate medium under sterile conditions at 20°C-35°C (e.g., 28-32°C for a sufficient amount of time, e.g. 40 to 150 hours (e.g., 60-90 hours) in an alternating electric field or a series of alternating electric fields as described above.

An exemplary set-up of the culture process is depicted in Fig. 1 (see above). An exemplary culture medium contains the following in per 1000 ml of sterile water: 20 g of mannitol, 50 µg of Vitamin C, 60 µg of Vitamin H, 40 µg of Vitamin B₁₂, 0.2 g of KH₂PO₄, 0.2 g of MgSO₄•7H₂O, 0.25 g of NaCl, 0.1 g of CaSO₄•2H₂O, 3.0 g of CaCO₃•5H₂O and 2.5 g of peptone. All vitamins are sterilized before added to the solution. Yeast cells of the desired strains are then added to the culture medium to form a mixture containing 1×10⁸ yeast cells per 1000 ml of culture medium. The yeast cells can be of any of the strains illustrated in Table 1. In one embodiment, the yeast cells are of the strain *Saccharomyces carlsbergensis Hansen* AS2.443. The mixture is then added to the apparatus of Fig. 1.

The activation process of the yeast cells involves the following steps: 1) maintaining the temperature of the activation apparatus at 20-35°C (e.g., 28-32°C), and culturing the yeast cells for 32-38 hours (e.g, 36 hours); 2) applying an electric field having a frequency of about 13103 MHz and a field strength of 240-280 mV/cm (e.g., about 257 mV/cm) for 12-18 hours (e.g., 14 hours); 3) maintaining the temperature of the activation apparatus at 28-32°C, culturing the yeast cells for 32-38 hours (e.g., 36 hours); 4) then applying an electric field having a frequency of about 13107 MHz and a field strength of 250-280 mV/cm (e.g., about 277 mV/cm) for 16-22 hours (e.g., 18 hours); 5) then applying an electric field having a frequency of about 13113 MHz and a field strength of 260-280 mV/cm (about 272 mV/cm) for 18-22 hours (e.g., 21 hours); 6) then applying an electric field having a frequency of about 13119 MHz and a field strength of 270-290 mV/cm (e.g., about 282 mV/cm) for 16-22 hours (e.g., 19 hours); 7) then applying an electric field having a frequency of about 13125 MHz and a field strength of 260-290 mV/cm (e.g., about 273 mV/cm) for 13-20 hours (e.g., 14 hours); and 7) finally lyophilizing the activated yeast cells to form a powder and storing the powder at 4 °C. Preferably, the concentration of the lyophilized yeast cells are more than 10¹⁰ cells/g.

### V. Acclimatization of Yeast Cells To the Gastric Environment

Because the yeast compositions of this invention must pass through the stomach before reaching the small intestine, where the effective components are released from these yeast cells, it is preferred that these yeast cells be cultured under acidic conditions to acclimatize the cells to the gastric juice. This acclimatization process results in better viability of the yeasts in the acidic gastric environment.

To achieve this, the yeast powder containing activated yeast cells can be mixed with an acclimatizing culture medium at 10 g (containing more than 10¹⁰ activated cells per gram) per 1000 ml. The yeast mixture is then cultured first in the presence of an alternating electric field having a frequency of about 13119 MHZ and a field strength of 350-380 mV/cm (e.g., about 372 mV/cm) at about 28 to 32°C for 36-42 hours (e.g., 40 hours). The resultant yeast cells are further incubated in the presence of an alternating electric field having a frequency of about 13125 MHZ and a field strength of 300-330 mV/cm (e.g., about 303 mV/cm) at about 28 to 32°C for 20-30 hours (e.g., 24 hours). The resulting acclimatized yeast cells are then either dried and stored in powder form (≥ 10¹⁰ cells/g) at room temperature or stored in vacuum at 0-4°C.

An exemplary acclimatizing culture medium is made by mixing 700 ml of fresh pig gastric juice and 300 ml of wild Chinese hawthorn extract. The pH of acclimatizing culture medium is adjusted to 2.5 with 0.1 M hydrochloric acid and 0.2 M potassium biphthalate. The fresh pig gastric juice is prepared as follows. At about 4 months of age, newborn Holland white pigs are sacrificed, and the entire contents of their stomachs are retrieved and mixed with 2000 ml of water under sterile conditions. The mixture is then allowed to stand for 6 hours at 4°C under sterile conditions to precipitate food debris. To prepare the wild Chinese hawthorn extract, 500 g of fresh wild Chinese hawthorn is dried under sterile conditions to reduce the water content (≤8%). The dried fruit is then ground (≥20 mesh) and added to 1500 ml of sterile water. The mixture is allowed to stand for 6 hours at 4°C under sterile conditions. The supernatant is collected to be used in the acclimatizing culture medium.

### VI. Manufacture of Yeast Compositions

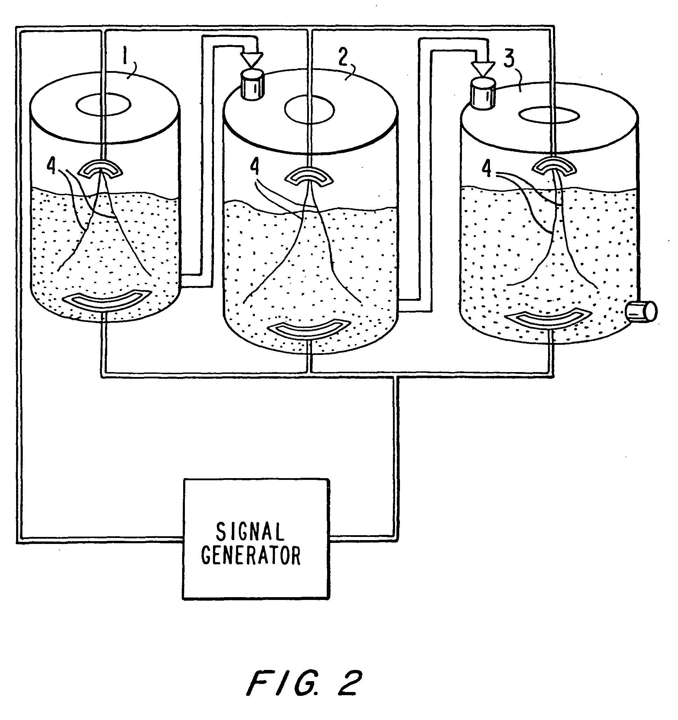

To prepare the yeast compositions of the invention, an apparatus depicted in Fig. 2 or an equivalent thereof can be used. This apparatus includes a first container (1), a second container (2), and a third container (3), each equipped with a pair of electrodes (4). One of the electrodes is a metal plate placed on the bottom of the containers, and the other electrode comprises a plurality of electrode wires evenly distributed in the space within the container to achieve even distribution of the electric field energy. All three pairs of electrodes are connected to a common signal generator.

The culture medium used for this purpose is a mixed fruit extract solution containing the following ingredients per 1000 L: 300 L of wild Chinese hawthorn extract, 300 L of jujube extract, 300 L of fruit extract from *Schisandra chinensis Baill* (wu wei zi), and 100 L of soy bean extracts. To prepare hawthorn, jujube and wu wei zi extracts, the fresh fruits are washed and dried under sterile conditions to reduce the water content to no higher than 8%. One hundred kilograms of the dried fruits are then ground (≥20 mesh) and added to 400 L of sterile water. The mixtures are stirred under sterile conditions at room temperature for twelve hours, and then centrifuged at 1000 rpm to remove insoluble residues. To make the soy bean extract, fresh soy beans are washed and dried under sterile conditions to reduce the water content to no higher than 8%. Thirty kilograms of dried soy beans are then ground into particles of no smaller than 20 mesh, and added to 130 L of sterile water. The mixture is stirred under sterile conditions at room temperature for twelve hours and centrifuged at 1000 rpm to remove insoluble residues. Once the mixed fruit extract solution is prepared, the solution is sterilized at 121°C for 30 minutes, and cooled to 40°C before use.

One thousand grams of the activated yeast powder prepared as described above (Section V, *supra)* is added to 1000 L of the mixed fruit extract solution, and the yeast solution is transferred to container (1) shown in Fig. 2. The yeast cells are then cultured in the presence of an alternating electric field having a frequency of about 13119 MHZ and a field strength of about 270-290 mV/cm (e.g., about 284 mV/cm) at 28-32°C under sterile conditions for 14 hours. The yeast cells are further incubated in an alternating electric field having a frequency of about 13125 MHZ and a field strength of 260-290 mV/cm (e.g., about 273 mV/cm). The culturing continues for another 10 hours.

The yeast culture is then transferred from the first container (1) to the second container (2) (if need be, a new batch of yeast culture can be started in the now available first container (1)), and subjected to an alternating electric field having a frequency of about 13119 MHZ and a field strength of 190-220 mV/cm (e.g., about 206 mV/cm) for 12 hours. Subsequently the frequency and field strength of the electric field are changed to about 13125 MHZ and 200-220 mV/cm (e.g., about 202 mV/cm), respectively. The culturing continues for another 10 hours.

The yeast culture is then transferred from the second container (2) to the third container (3), and subjected to an alternating electric field having a frequency of about 13119 MHZ and a field strength of 80-100 mV/cm (e.g., about 94 mV/cm) for 18 hours. Subsequently the frequency and field strength of the electric field are changed to about 13125 MHZ and 60-80 mV/cm (e.g., about 73 mV/cm), respectively. The culturing continues for another 14 hours.

The yeast culture from the third container (3) can then be packaged into vacuum sealed bottles for use as a dietary supplement. The dietary supplement can be taken 3-4 times daily at 30-60 ml each time for a period of three months (10-30 minutes before meals and at bedtime). If desired, the final yeast culture can also be dried within 24 hours and stored in powder form.

In one embodiment, the compositions of the invention can also be administered intravenously or peritoneally in the form of a sterile injectable preparation. Such a sterile preparation is prepared as follows. A sterilized health drink composition is first treated under ultrasound (1000 Hz) for 10 minutes and then centrifuged at 4355 rpm for another 10 minutes. The resulting supernatant is adjusted to pH 7.2-7.4 using 1 M NaOH and subsequently filtered through a membrane (0.22 µm for intravenous injection and 0.45 µm for peritoneal injection) under sterile conditions. The resulting sterile preparation is submerged in a 35-38 °C water bath for 30 minutes before use.

### VII. Examples

The following examples are meant to illustrate the methods and materials of the present invention. Suitable modifications and adaptations of the described conditions and parameters which are obvious to those skilled in the art are within the spirit and scope of the present invention.

The activated yeast compositions used in the following experiments were prepared as described above, using *Saccharomyces carlsbergensis Hansen* AS 2.443 cultured in the presence of an alternating electric field having the electric field frequency and field strength exemplified in the parentheses following the recommended ranges in Section IV, *supra.* Control yeast compositions were those prepared in the same manner except that the yeast cells were cultured in the absence of EMFs. Unless otherwise indicated, the yeast compositions and the corresponding controls were admitted to the animals via intragastric feeding.

### Example 1: Enkephalin Assay of Rats with Hypertension

The composition of this invention can regulate the metabolism of enkephalin in rats with hypertension. In general, endorphins function as neurotransmitters and regulators of neurons and are involved in the regulation of blood vessels. The increase in enkephalin decreases the activity of the sympathetic nerve, thereby alleviating the hypertension. In this experiment, enkephalin in the sample competed with ¹²⁵I radiolabeled enkephalin for an anti-enkephalin antibody. After the reaction reached equilibrium, rabbit anti-IgG (r_{G} ) and sheep anti-rabbit antibodies were used to separate the enkephalin-antibody complex and the free enkephalin (F). The r_{G} and AAb were in the precipitate, and the free enkephalin appeared in the supernatant. The radioactive signal in the precipitate was measured.

Thirty 10-12 month old Wistar rats that weighed about 200 g were selected for the assay. The blood pressures of the rats were monitored for three days. Then, hypertension in the rats was induced by administering daily, by subcutaneous injection, 4 mg of testosterone propionate for 14 days, until the blood pressure of the rats increased to 1.3 KPa. The rats with hypertension were separated into groups A, B and C. Each rat in groups A, B and C was administered daily 2 ml of the activated yeast composition, the control yeast composition, and saline, respectively for 12 weeks. Ten healthy Wistar rats were assigned to group D, the non-treatment control. Each rat in group D was given daily 2 ml of saline for 12 weeks.

After 12 weeks, the rats were sacrificed. The brain of each rat was taken out and placed in boiling saline for 4 minutes. Then, the brain was dissected into sections of the brain stem, hypothalamus and striatum along their natural boundaries. The sections were weighed and mixed with 3 ml of 0.1 M HCl. Afterwards, 0.3 ml of 1 M NaOH and 0.7 ml of 0.5 M PELH buffer (pH 7.6) were added to the mixture. The PELH buffer was prepared by mixing 0.1 mol/L of phosphate-buffered saline (PBS) (pH 7.6), 0.003 mol/L of ethylenediamine tetraacetic acid disodium salt dihydrate, 1mg/dl bacteriolysozyme and 0.02 mg/dl chlorhexidinum (Hibitane). Finally, PELH buffer was added to the mixture to obtain a solution of 5 ml. The solution was centrifuged at 3300 g for 20 minutes. The supernatant was diluted with PELH buffer, and 0.1 ml of the diluent to a volume of 0.5 ml. The diluted supernatant was used to perform the immunoradioassay for met-enkephalin (MEK) and leu-enkephalin (LEK).

The results in Table 2 illustrate that for the control groups with healthy rats (group D), the MEK and LEK values are high. For the rats with hypertension, the MEK and LEK values for the group treated with the activated yeast composition (group A) were substantially higher than groups treated with control yeast composition or saline (group B or C) . Therefore, the activated yeast composition of this invention has the ability to alleviate hypertension.

**Table 2**

| Group | Animal number | MEK in brain stem (pg/mg) | | MEK in hypothalamus (pg/mg) | | LEK in brain stem (pg/mg) | | LEK in hypothalamus (pg/mg) | |
|---|---|---|---|---|---|---|---|---|---|
| | | before 12 weeks | after 12 weeks | before 12 weeks | after 12 weeks | before 12 weeks | after 12 weeks | before 12 weeks | after 12 weeks |
| A | 2x10 | 57.43 ± 12.74 | 116 ± 14.67 | 123.54 ± 41.43 | 304.63 ± 38.68 | 52.12 ± 12.36 | 102.43 ± 11.54 | 119.67 ± 34.14 | 263.72 ± 34.17 |
| B | 2x10 | 61.53 ± 14.56 | 67.23 ± 16.73 | 128.45 ± 47.67 | 136.87 ± 52.47 | 58.54 ± 16.43 | 63.52 ± 17.73 | 121.38 ± 43.31 | 134.49 ± 51.37 |
| C | 2x10 | 59.36 ± 11.71 | 63.76 ± 17.89 | 113.46 ± 43.57 | 132.34 ± 56.78 | 54.57 ± 17.86 | 59.74 ± 13.46 | 124.47 ± 42.37 | 133.84 ± 32.41 |
| D | 2x10 | 121.26 ± 16.33 | 120.14 ± 19.71 | 308.21 ± 61.22 | 311.53 ± 57.34 | 112.76 ± 24.45 | 109.67 ± 19.78 | 277.54 ± 56.52 | 287.42 ± 48.97 |

### Example 2: Electroencephalogram of Rabbit Cerebral Cortex

The central nervous system can be studied by analyzing the electroencephalogram of the brain. While an increase in the low frequency EEG power spectra indicates that the central nervous system is calm, a decrease indicates that the central nervous system is excited. For the high frequency EEG power spectra this correlation is reversed. The following electroencephalogram experiment analyzes the calming effect of the yeast composition.

Twenty Angola rabbits were divided into groups of 5. The rabbits were locally anesthetized with 2% procaine hydrochloride on the top of the head. The skull of the rabbit was cleaned after removing the skin. Then, surgery was carried out to place electrodes at positions A₂, P₄, R₄, L₄ and H₂. After half an hour, the electrocortical signal of the cerebral cortex at the right and left sides of the forehead was recorded. At the same time, the electrocortical signal was entered into a computer through a direct current amplifier at a sampling speed of 8 bit/10 seconds for 102400 seconds. The electrocortical signal measured before treatment was used as a reference. The signal was measured twice within 30-60 minutes after the operation.

Sixty minutes after the operation, rabbits in Group A were administered 8 ml of the activated yeast composition per kg body weight. Group B was treated with the control yeast composition. Group C was treated with 2.0 mg of diazepam per kg body weight, and group D was treated with 8 ml of saline per kg body weight.

After treatment, the electrocortical signal was measured three times within sixty minutes, and the data were entered into a computer and stored on a disk. The self-recording power spectra was calculated by performing a Fast Fourier Transform on the electrocortical signal. The results of the experiment are illustrated in Table 3.

**Table 3**

| Group | Animal number | low frequency EEG power spectra before treatment (mw) | low frequency EEG power spectra after treatment (mw) | change in low frequency EEG power spectra (%) |
|---|---|---|---|---|
| A | 5 | 208.25 | 1242.57 | 496.7 |
| B | 5 | 209.58 | 210.23 | 3.1 |
| C | 5 | 204.98 | 1002.37 | 388.8 |
| D | 5 | 251.52 | 241.46 | -4.0 |

As illustrated above, for control groups B and D, after treatment, the low frequency EEG power spectra only changed minimally. For group C, which was treated with the drug diazepam, a suppressor of the central nervous system, the low frequency EEG power spectra was substantially increased. For group A, which was treated with the activated yeast composition, the low frequency EEG power spectra was also substantially increased. Further, while drowsiness was observed in animals treated with diazepam, the activated yeast composition did not produce such an effect.

While a number of embodiments of this invention have been set forth, it is apparent that the basic constructions may be altered to provide other embodiments which utilize the compositions and methods of this invention.

## Claims

1. A composition comprising a plurality of yeast cells, wherein said plurality of yeast cells are **characterized by** an increase in their capability to increase the level of met-enkephalin or leu-enkephalin in a brain cell or brain tissue of a mammal as a result of having been cultured in the presence of an alternating electric field having a frequency in the range of 13050 to 13150 MHz and a field strength in the range of 20 to 400 mV/cm, as compared to yeast cells not having been so cultured.

2. A composition comprising a plurality of yeast cells, wherein said plurality of yeast cells are **characterized by** an increase in their capability to substantially increase the low frequency EEG power spectra of the brain of a mammal as a result of having been cultured in the presence of an alternating electric field having a frequency in the range of 13050 to 13150 MHz and a field strength in the range of 20 to 400 mV/cm, as compared to yeast cells not having been so cultured.

3. The composition of claim 1 or 2, wherein the range of the frequency is 13100-13150 MHz.

4. The composition of any of claims 1 to 3, wherein the range of the field strength is 50-300 mV/cm.

5. The composition of any of claims 1 to 4, wherein said yeast cells are of the species selected from *Saccharomyces sp, Schizosaccharomyces pomne Lindner, Saccharmyces sake Yabe, Saccharomyces* *urarum Beijer, Saccharomyces rouxii Boutroux, Saccharomyces cerevisiae Hansen Var. ellipsoideus, Saccharomyces carlsbergensis Hansen, Rhodotorula aurantiaca Lodder and Saccharomyces cerevisiae Hansen.*

6. The composition of claim 1 or 2, wherein said yeast cells are of the strain deposited at the China General Microbiological Culture Collection Center with an accession number selected from the group consisting of AS2.443, AS2.501, AS2.502, AS2.503, AS2.504, AS2.535, AS2.558, AS2.560, AS2.561 and AS2.562.

7. The composition of claim 6, wherein said strain is AS2.443.

8. The composition of any of claims 1 to 7, wherein the composition is in the form of a tablet, powder or healthdrink.

9. The composition of claim 8, wherein the composition is in the form of a healthdrink.

10. A method of preparing a yeast composition, comprising culturing a plurality of yeast cells in the presence of an alternating electric field having a frequency in the range of 13050 to 13150 MHz and a field strength in the range of 100 to 600 mV/cm, wherein said plurality of yeast cells are **characterized by** an increase in their capability to increase the level of met-enkephalin or leu-enkephalin in a brain cell or brain tissue of a mammal as a result of said culturing as compared to yeast cells not having been so cultured.
